# EUROPÄISCHE PATENTSCHRIFT

(11) **EP 3 122 294 B1**
(45) Veröffentlichungstag und Bekanntmachung des Hinweises auf die Patenterteilung: **12.12.2018**
(21) Anmeldenummer: 15709625.6
(22) Anmeldetag: 24.02.2015
(51) Int. Cl.: A61F 5/56

(54) **OKKLUSIONSSCHIENENANORDNUNG**
OCCLUSION SPLINT ASSEMBLY
DISPOSITIF INTRAORAL D'OCCLUSION

(30) Priorität: 28.03.2014 DE 102014104408
(43) Veröffentlichungstag der Anmeldung: 01.02.2017
(73) Patentinhaber: Hofmann, Konrad, 97291 Thüngersheim (DE)
(72) Erfinder: Hofmann, Konrad, 97291 Thüngersheim (DE)
(74) Vertreter: advotec.
(86) Internationale Anmeldenummer: PCT/EP2015/053792
(87) Internationale Veröffentlichungsnummer: WO 2015/144374

(56) Entgegenhaltungen:
- WO-A1-2013/143511
- US-A- 4 527 975
- US-A1- 2005 028 826
- US-A1- 2006 019 213

## Beschreibung

Die Erfindung betrifft eine Okklusionsschienenanordnung, insbesondere zur Schlafapnoe-Therapie, mit einer maxillaren und einer mandibularen Miniplastschiene gemäß dem Oberbegriff des Anspruchs 1. Insbesondere zur Schlafapnoe-Therapie sind aus dem Stand der Technik verschiedenste Ausführungsformen von Okklusionsschienenanordnungen bekannt. Diese zielen grundsätzlich darauf ab, die Stellung des Oberkiefers relativ zum Unterkiefer dahingehend zu beeinflussen, dass im tiefen Schlaf ein Zurücksacken des Unterkiefers sowie des Zungenmuskels und des Gaumensegels in zulässigem Maße begrenzt wird, so dass die Atemwege offengehalten werden.

Eine für diese Aufgabe besonders vorteilhafte Ausführungsform offenbart die Druckschrift WO 2013/143511 A1, die als nächstliegender Stand der Technik angesehen ist. Diese umfasst eine maxillare Miniplastschiene zur Anordnung auf der maxillaren Zahnreihe sowie eine mandibulare Miniplastschiene zur Anordnung auf der mandibularen Zahnreihe. Die beiden Miniplastschienen weisen jeweils Positionierungsmittel auf, anhand derer die Position der Miniplastschienen zueinander festlegbar ist. Die beiden Miniplastschienen sind hierbei nicht aneinander fixiert, sondern der Verwender kann beim Tragen der Miniplastschienen den Unterkiefer weitestgehend ungehindert bewegen. Beim Aufeinandertreffen der gegenüberliegenden Anlageflächen der Miniplastschienen erfolgt die gewünschte Positionierung der mandibularen Miniplastschiene relativ zur maxillaren Miniplastschiene anhand der jeweiligen Positionierungsmittel.

Hierzu weist die maxillare Miniplastschiene im molaren Bereich auf linker und rechter Seite integral die Positionierungsmittel auf, wobei jeweils eine nach außen weisende Führungsfläche und eine nach vorne weisende Anlagefläche vorhanden sind. Demgegenüber besitzt die mandibulare Miniplastschiene an entsprechend übereinstimmender Position Positionierungsmittel in der Art einer Finne, welche auf der Anlagefläche der mandibularen Miniplastschiene positioniert ist. Das Besondere an der zuvor genannten Ausführungsform im Gegensatz zur sonst üblichen Okklusionsschienenanordnung zur Schlafapnoe-Therapie ist die Auswechselbarkeit der Positionierungsmittel der mandibularen Miniplastschiene. In der Miniplastschiene ist auf der linken und rechten Seite im molaren Bereich ein über der Anlagefläche hervorstehender Zentrierstift verankert. An diesem ist jeweils ein auswechselbares Positionierungsmittel auswechselbar befestigt.

Diese bereits vorteilhafte Ausführungsform ermöglicht ein schnelles und unkompliziertes Wechseln der Positionierungsmittel, wodurch eine Veränderung der Lage der mandibularen Miniplastschiene relativ zur maxillaren Miniplastschiene in der Längsrichtung (X) ermöglicht wird.

Wenngleich mit der bekannten Ausführungsform bereits eine besonders vorteilhafte Okklusionsschienenanordnung geschaffen ist, welche sowohl komfortabel im Trageverhalten als auch wirkungsvoll in der Schlafapnoe-Therapie ist, zeigten sich jedoch in der Umsetzung, insbesondere bei der Abstimmung vom Zentrierstift und der im auswechselbaren Positionierungsmittel vorhandenen komplementären Zentrieraufnahme, Probleme hinsichtlich der Zuverlässigkeit des sicheren Halts des montierten auswechselbaren Positionierungsmittels. In bekannten Ausführungsformen trat mitunter das Problem auf, dass das auswechselbare Positionierungsmittel nicht hinreichend an dem Zentrierstift der Miniplastschiene haftete und bei der Handhabung ungewollt abfiel. Im Gegensatz dazu wurde vielfach beobachtet, dass das Abnehmen des auswechselbaren Positionierungsmittels mitunter erheblich erschwert war, da die gewählten Reibkräfte zwischen Zentrierstift und Zentrieraufnahme unkontrolliert einen zu großen Wert angenommen hatten.

Die US 2006/019213 beschreibt eine Okklusionsschienenanordnung, bei der eine Rastverbindung zur Befestigung der Positionierungsmittel an der Okklusionsschienenanordnung verwendet wird.

Die US 2005/028826 beschreibt eine Okklusionsschienenanordnung, bei der eine Schraubverbindung zur Befestigung der Positionierungsmittel an der Okklusionsschienenanordnung verwendet wird.

Aufgabe der vorliegenden Erfindung ist es daher, eine Verbesserung der bekannten Ausführungsform, insbesondere hinsichtlich der Fixierung der Positionierungsmittel, zu schaffen.

Die gestellte Aufgabe wird durch eine erfindungsgemäße Ausführungsform nach der Lehre des Anspruchs 1 gelöst.

Vorteilhafte Ausführungsformen sind Gegenstand der Unteransprüche.

Die Okklusionsschienenanordnung dient insbesondere zur Schlafapnoe-Therapie. Wenngleich somit die vorliegende Ausführungsform einer Okklusionsschienenanordnung primär zur Therapierung des Schlafapnoe-Syndroms vorgesehen ist, so ist es jedoch unbenommen, die erfindungsgemäße Ausführungsform ebenso für eine anderweitige Okklusionsschienenanordnung einzusetzen. Hierbei kann die Okklusionsschienenanordnung ebenso vorgesehen sein zur Behandlung von Gebissfehlstellungen oder als Aufbissschiene zur Verhinderung eines nächtlichen Knirschens oder dergleichen.

Die gattungsgemäße Okklusionsschienenanordnung weist eine auf der maxillaren Zahnreihe anordenbare maxillare Miniplastschiene sowie eine auf der mandibularen Zahnreihe anordenbare mandibulare Miniplastschiene auf. Verwendungsgemäß kann hierbei die maxillare Miniplastschiene gegen die mandibulare Miniplastschiene zur Anlage gebracht werden. Hierbei weisen die Miniplastschienen gegenüberliegende Anlageflächen auf, welche im Wesentlichen innerhalb der Okklusionsebene gegeneinander zur Anlage gebracht werden können. In besonders vorteilhafter Weise sind die Anlageflächen hierbei eben ausgeführt, wodurch einerseits eine geringe Bauhöhe zwischen den Zahnreihen bei geschlossenem Mund erzielt wird und zugleich eine Veränderung der Stellung der Miniplastschienen zueinander innerhalb der Ebene der Anlagefläche möglich ist.

Zur Positionierung der Miniplastschienen zueinander weist die maxillare Miniplastschiene zumindest ein maxillares Positionierungsmittel sowie die mandibulare Miniplastschiene zumindest ein mandibulares Positionierungsmittel auf. Hierbei ist die Art, Ausführung und Anordnung der Positionierungsmittel an den Miniplastschienen zunächst unerheblich. Zumindest erfordert die Positionierung der Miniplastschienen relativ zueinander einen Formschluss zwischen dem maxillaren und mandibularen Positionierungsmittel, wodurch die Relativstellung der Miniplastschienen zueinander in einer Längsrichtung und/oder in einer Querrichtung definierbar ist.

Diesbezüglich ist es gleichfalls zunächst unerheblich, ob der Formschluss beispielsweise vor einem Einsetzen auf den Zahnreihen bereits außerhalb des Munds durch Fügen der Miniplastschienen aneinander hergestellt wird, oder ob ein Formschluss erst in dem Fall auftritt, wenn die auf den Zahnreihen aufgesetzten Miniplastschienen durch ein Schließen des Munds in Kontakt zueinander gebracht werden. Zumindest wird spätestens beim Schließen des Gebisses durch Anlage der Miniplastschienen aneinander ein entsprechender Formschluss zwischen den Positionierungsmitteln bewirkt, so dass die relative Stellung der Miniplastschienen zueinander definiert wird. Diese relative Stellung kann hierbei sowohl die Längsrichtung, d.h. eine Erstreckung nach vorne bzw. nach hinten aus Sicht des Patienten, oder alternativ oder zugleich eine relative Positionierung in einer Querrichtung umfassen. Diesbezüglich ist es für die erfindungsgemäße Ausführungsform weiterhin zunächst unerheblich, ob hierbei ein geringfügiges Spiel vorgesehen ist.

Hinsichtlich der benannten Richtungen ist auszuführen, dass aus Sicht des Patienten sich die Längsrichtung entlang einer Linie von hinten nach vorne erstreckt, wobei die Hochrichtung näherungsweise senkrecht zur Okklusionsebene liegt und des Weiteren die Querrichtung entsprechend senkrecht auf der Hochrichtung sowie senkrecht zur Längsrichtung liegt, d.h. aus Sicht des Patienten von links nach rechts (bzw. umgekehrt) verläuft.

Gattungsgemäß ist hierbei eine Anordnung der Positionierungsmittel an den jeweiligen Miniplastschienen auf der linken und auf der rechten Seite im molaren Bereich an der Miniplastschiene vorgesehen. Das heißt, dass je Miniplastschiene zumindest zwei Positionierungsmittel vorhanden sein müssen. Dies ist dahingehend besonders vorteilhaft, dass der für die Positionierungsmittel erforderliche Bauraum bzw. der Platzbedarf im entsprechend molaren Bereich ohne besondere Beeinträchtigung des tragenden Patienten in Anspruch genommen werden kann. Hierbei ist es vorteilhaft (jedoch nicht zwingend), wenn die Positionierungsmittel sich auf der Außenseite der Zahnreihe befinden. Das heißt, dass sich die Positionierungsmittel im Backenbereich zwischen Zähnen und Wange befinden.

Wesentlich für die gattungsgemäße Ausführungsform zur Ermöglichung einer Veränderung der Stellung der Miniplastschienen in Längsrichtung weist die maxillare und/oder mandibulare Miniplastschiene zumindest zwei aus der Anlagefläche herausstehende Zentrierstifte auf. Wenngleich es zunächst unerheblich ist, ob die maxillare Miniplastschiene oder die mandibulare Miniplastschiene die Zentrierstift aufweist, ist es jedoch vorteilhaft, wenn die Anordnung der Zentrierstifte an der mandibularen Miniplastschiene erfolgt. Hierbei sind die Zentrierstifte auf der linken Seite sowie auf der rechten Seite im molaren Bereich in der Miniplastschiene zu verankern. Oberhalb der Anlagefläche erstreckt sich der Zentrierstift näherungsweise in Hochrichtung.

Zur Okklusionsschienenanordnung gehören weiterhin auswechselbare Positionierungsmittel, welche zu den jeweiligen Zentrierstiften komplementäre Zentrieraufnahmen aufweisen. Entsprechend dem Vorhandensein eines linken Zentrierstifts sowie eines rechten Zentrierstifts ist somit zumindest ein linkes auswechselbares Positionierungsmittel und ein rechtes auswechselbares Positionierungsmittel erforderlich. Entsprechend der bekannten Ausführungsform werden auswechselbare Positionierungsmittel derart ausgeführt, dass diese an den an der Miniplastschiene befindlichen Zentrierstiften befestigt werden können. Hierbei ist vorgesehen, dass die auswechselbaren Positionierungsmittel auf der Anlagefläche der zugehörigen Miniplastschiene zur Anlage kommen. Zur Ermöglichung verschiedener relativer Stellungen zwischen der mandibularen und der maxillaren Miniplastschiene ist weiterhin vorgesehen, dass das am Zentrierstift befestigte Positionierungsmittel ausgetauscht werden kann durch ein anderes, in der Geometrie abweichendes Positionierungsmittel. Das heißt, dass jedes Positionierungsmittel, welches zur Befestigung am Zentrierstift bestimmt ist, eine individuelle relative Stellung der Miniplastschienen zueinander bewirken kann.

In einfacher und vorteilhafter Ausführung sind die Anlageflächen vollständig eben ausgeführt, so dass die Okklusionsebene auch die Anlagefläche für die auswechselbaren Positionierungsmittel darstellt. Jedoch ist dies keine zwingende Ausführungsform. Ebenso ist es möglich, die Anlagefläche ausgehend von der ebenen Gestalt im Bereich der Auflage der auswechselbaren Positionierungsmittel geringfügig zu kippen, so dass beispielsweise bei einem geneigt zur Hochachse ausgerichteten Zentrierstift die Anlagefläche im Bereich des auswechselbaren Positionierungsmittel eine lokale Winkelabweichung zur restlichen Anlagefläche aufweist. D.h. die Anlagefläche im Bereich der auswechselbaren Positionierungsmittel wird übereinstimmend mit der Winkelabweichung des Zentrierstifts von der Hochrichtung in Richtung der Querrichtung geneigt ausgeführt.

Entsprechend der Möglichkeit, das am Zentrierstift befestigte Positionierungsmittel austauschen zu können, folgt hieraus zwangsläufig, dass ebenso lediglich ein einzelnes Positionierungsmittel je Zentrierstift vorhanden sein kann, welches zwar prinzipiell austauschbar ist, hierzu jedoch kein zweites abweichendes Positionierungsmittel zum Austausch zur Verfügung steht.

Erfindungsgemäß wird die bekannte Ausführungsform dadurch erheblich verbessert, dass der Zentrierstift eine Rastnase und die Zentrieraufnahme eine Rastvertiefung aufweist, wobei die Rastnase durch Einrasten in der Rastvertiefung eine Fixierung des auswechselbaren Positionierungsmittels an der Miniplastschiene bewirkt.

Durch den Einsatz einer Rastverbindung zwischen der Miniplastschiene mit dem Zentrierstift und dem Positionierungsmittel mit der Zentrieraufnahme wird eine sichere Befestigung der auswechselbaren Positionierungsmittel erreicht. Wenngleich sich hierdurch die Demontage des auswechselbaren Positionierungsmittels aufwendiger gestaltet als es bei vorheriger Ausführungsform bekannt war, so ist das vorherige Problem der Abstimmung zwischen Zentrierstift und Zentrieraufnahme weitestgehend gelöst. Nunmehr wird durch den Einsatz einer Rastverbindung ein kontrollierter Zustand geschaffen, so dass sich die Haltekräfte des Positionierungsmittels an der Miniplastschiene im Wesentlichen von der Auslegung der Rastverbindung bestimmen. Die Herstellung des Zentrierstifts kann prozesssicher in engen Toleranzen erfolgen. Darüber hinaus wurde festgestellt, dass auch die Zentrieraufnahme im relevanten Bereich der Rastvertiefung erheblich einfacher in kontrollierten Toleranzen hergestellt werden kann, als es beim Positionierungsmittel mit der zuvor engen Tolerierung der Zentrieraufnahme zur Gewährleistung der Reibverbindung der Fall war. Diesbezüglich bedarf es nunmehr lediglich der genauen Tolerierung der Rastvertiefung im Bereich der Anlagefläche der Rastnase.

Zur Realisierung des Zentrierstifts sowie zu dessen Befestigung an der Miniplastschiene ist erfindungsgemäß vorgesehen, dass diese einen in der Miniplastschiene eingegossenen Sockelabschnitt und einen starr über der Anlagefläche überstehenden Befestigungsabschnitt aufweisen. Darüber hinaus ist es zur Realisierung der Rastfunktion besonders vorteilhaft, wenn am Befestigungsabschnitt ein elastisch verformbarer Verbindungsabschnitt angeformt ist, an dessen freiem Ende sich die Rastnase befindet. Somit wird die Rastfunktion realisiert, indem beim Fügen des Positionierungsmittels an der Miniplastschiene eine elastische Verformung des Verbindungsabschnitts stattfindet, wobei die Rastnase im Folgenden in die Rastvertiefung unter teilweiser bzw. weitgehender Rückverformung des Verbindungsabschnitts eingreifen kann.

Bei Realisierung eines Zentrierstifts mit Rastnase ist es insbesondere aus Kostengründen besonders vorteilhaft, wenn dieser einstückig ausgeführt ist. Darüber hinausgehend ist es vorteilhaft, zur Herstellung ein Metallblech zu verwenden, welches vorteilhafterweise durch Laserschneiden bearbeitet wird. Somit kann ein Zentrierstift hoher Genauigkeit hergestellt werden, wobei insbesondere die kleinen Abmessungen des Zentrierstifts zur Verwendung in einer Miniplastschiene zu berücksichtigen sind. Darüber hinaus ist es besonders vorteilhaft, wenn es sich bei dem Metallblech um einen Federstahl handelt, so dass die Rastfunktion vorteilhaft vom Zentrierstift realisiert wird und hierbei eine langlebige Verwendungsdauer ohne Einbußen in der Rastkraft garantiert werden kann.

Insbesondere in Verbindung mit der Verwendung eines Metallblechs, besonders aus Federstahl, wird eine besonders zuverlässige Rastfunktion durch Realisierung eines elastischen Verbindungsabschnitts ermöglicht. Demgegenüber ist in vorteilhafter Weise beim auswechselbaren Positionierungsmittel kein elastisch verformbarer Bereich erforderlich, sondern vielmehr kann eine im Wesentlichen sich nicht verformende Gestaltung der Zentrieraufnahme mit Rastvertiefung gewählt werden.

Demgegenüber wäre es jedoch ebenso denkbar, aber nicht erfindungsgemäß, den Zentrierstift insgesamt starr und unverformbar auszuführen, so dass die Rastnase beim Fügen des auswechselbaren Positionierungsmittels an der Miniplastschiene mit Aufschieben der Zentrieraufnahme auf den Zentrierstift nicht verformt wird bzw. nicht ausweicht. Hierbei wird zur Realisierung einer Rastfunktion jedoch eine Verformbarkeit des auswechselbaren Positionierungsmittels gefordert, so dass sich die Rastvertiefung nach zwischenzeitlicher Verformung des auswechselbaren Positionierungsmittels im Bereich der Zentrieraufnahme in die Rastvertiefung einfügen kann. Diese Ausführungsform ist jedoch lediglich in den Fällen von besonderem Interesse, wenn das auswechselbare Positionierungsmittel lediglich sehr selten oder nur einmalig demontiert werden muss oder aber als Wegwerfartikel ausgeführt wird und vielfach durch ein identisches Positionierungsmittel ersetzt wird.

Darüber hinaus ist alternativ zur erfindungsgemäßen Ausführung eine Befestigung des auswechselbaren Positionierungsmittels am Zentrierstift durch eine Rastfunktion realisierbar, bei der der Zentrierstift eine Rastausnehmung und das auswechselbare Positionierungsmittel eine Rastnase aufweist. Insbesondere bei einem aus einem Metallblech gebildeten Zentrierstift lässt sich ohne weiteres eine Rastvertiefung durch eine seitliche oder mittige Ausnehmung erzeugen. Demgegenüber benötigt das auswechselbare Positionierungsmittel im Bereich der Zentrieraufnahme eine in die Zentrieraufnahme hineinragende Rastnase. Diese ist beispielsweise bei einem als Kunststoff-Spritzgussteil hergestellten auswechselbaren Positionierungsmittel mit Hilfe eines Schiebers im Werkzeug ebenso ohne weiteres realisierbar. Zu bedenken ist hierbei - wie auch bei vorheriger Ausführung - dass beim Fügen des auswechselbaren Positionierungsmittels auf die Miniplastschiene eine elastische Verformung des auswechselbaren Positionierungsmittels insgesamt durch die in die Zentrieraufnahme hineinragende Rastnase erfolgt.

Zur Realisierung eines elastisch verformbaren Verbindungsabschnitts ist es in einer ersten Variante möglich, die Anbindung an dem starren Befestigungsabschnitt unmittelbar oberhalb der Anlagefläche vorzusehen. Dementsprechend befindet sich die Rastnase am von der Anlagefläche fernen Ende des Zentrierstifts. Diese Variante erfordert jedoch beim Fügen des auswechselbaren Positionierungsmittels ein tiefes Eintauchen der Rastnase in die Zentrieraufnahme bei Verformung des Verbindungsabschnitts und erschwert somit den Montageprozess.

Demgegenüber ist es besonders vorteilhaft, wenn der elastisch verformbare Verbindungsabschnitt am von der Anlagefläche entfernten Ende des starren Befestigungsabschnitts angebunden ist. Somit beginnt der elastisch verformbare Verbindungsabschnitt quasi am freien Ende des Zentrierstifts. Im Folgenden erstreckt sich der elastisch verformbare Verbindungsabschnitt in Richtung zur Anlagefläche entlang des Befestigungsabschnitts. Diese vorteilhafte Ausführungsform begünstigt die Montage des auswechselbaren Positionierungsmittels, da sich in diesem Fall die Rastnase nahe der Anlagefläche befindet und somit beim Fügen der Zentrieraufnahme auf den Zentrierstift von der Rastnase lediglich ein kurzer Abschnitt unter Verformung des elastischen Verbindungsabschnitts überwunden werden muss, bis die Rastnase in die zugehörige Rastvertiefung eintauchen kann.

Zur Verbesserung der elastischen Verformung des Verbindungsabschnitts mit einer hinreichend großen Überschneidung der Rastnase beim Eintauchen in die Zentrieraufnahme bei gleichzeitig möglichst geringen Verformungskräften ist es vorteilhaft, wenn der Verbindungsabschnitt möglichst lang ausgeführt wird. Daher ist es weiterhin besonders vorteilhaft, wenn sich der elastische Verbindungsabschnitt derartig am starren Befestigungsabschnitt anschließt, dass sich dieser zunächst um ein freies Auge im Halbkreis um 170° +/- 10° erstreckt. Besonders vorteilhaft ist eine Formgebung, bei der der Verbindungsabschnitt zunächst über einen Winkel von 175° +/- 5° um ein freies Auge geführt wird und sodann sich näherungsweise parallel zum Befestigungsabschnitt erstreckt. Somit kann die Länge des elastisch verformbaren Verbindungsabschnitts effektiv vergrößert werden, ohne dass eine nennenswerte Vergrößerung der Länge des Zentrierstifts erforderlich ist.

Weiterhin ist es vorteilhaft, wenn sich im nicht eingerasteten Zustand ein Spalt zwischen dem Befestigungsabschnitt und dem Verbindungsabschnitt ausgehend vom Ende des Befestigungsabschnitts aufweitet. In der vorteilhaften Ausführungsform mit zunächst im Halbkreis geführtem Verbindungsabschnitt um ein freies Auge wird der Spalt ausgehend vom freien Auge zum Ende des Verbindungsabschnitts geführt. Das Aufweiten des Spalts zwischen Verbindungsabschnitt und Befestigungsabschnitt gewährleistet die elastische Verformbarkeit des Verbindungsabschnitts mit Annäherung an den Befestigungsabschnitt beim Fügen des auswechselbaren Positionierungsmittels an der Miniplastschiene.

Die vorteilhafte Gestaltung des elastisch verformbaren Verbindungsabschnitts mit seiner Erstreckung entlang des Befestigungsabschnitts unter Berücksichtigung von dessen Rastfunktion führt bei nicht eingerastetem Zustand zu einer sich aufweitenden Gestalt des Zentrierstifts ausgehend vom Anbindungspunkt des Verbindungsabschnitts an den Befestigungsabschnitt. D.h. in der vorteilhaften Ausführung weitet sich der Zentrierstift ausgehend von seinem freien Ende in Richtung der Anlagefläche auf. Hingegen verlaufen bei an der Miniplastschiene befestigtem Positionierungsmittel vorteilhaft eine vordere Fläche am Befestigungsabschnitt und eine hintere Fläche am Verbindungsabschnitt im Wesentlichen parallel zueinander, wobei der elastische Verformungsabschnitt entsprechend ausgehend vom unverformten Zustand elastisch verformt ist und die Rastnase unter Spannung in der Rastvertiefung hält. Folglich stellt sich eine hierzu komplementäre Zentrieraufnahme als quasi geradlinige Tasche ausgehend von der Anlagefläche dar. Geringfügige Winkelabweichungen zur Berücksichtigung einer zur Realisierung der Zentrieraufnahme herstellungsbedingt erforderlichen Entformungsschräge von beispielsweise 1° bis 2° sind diesbezüglich unerheblich.

Insbesondere begünstigt eine geradlinige Gestaltung der Zentrieraufnahme (ohne Betrachtung der Rastvertiefung) und des Zentrierstifts im eingerasteten Zustand eine sichere Fixierung des Positionierungsmittels an der Miniplastschiene.

In einer alternativen Ausführungsform wird der elastisch verformbare Verbindungsabschnitt nicht ausgehend vom Ende des Befestigungsabschnitts mit einem Bogen ausgeführt, sondern erstreckt sich vom Ende des Befestigungsabschnitts unmittelbar in Richtung zur Anlagefläche. Hierbei ist es besonders vorteilhaft, wenn sich der Verbindungsabschnitt im nicht eingerasteten Zustand ausgehend von dessen Anfang am Ende des Befestigungsabschnitts zunehmend in der Querrichtung vom Befestigungsabschnitt abhebt. D.h. der Verbindungsabschnitt wird elastisch in der Querrichtung ausgelenkt, wobei es zunächst unerheblich ist, zu welcher Seite die Auslenkung erfolgt. Die Stabilität des Befestigungsabschnitts sowie die Zentrierungsfunktion des Zentrierstifts für das auswechselbare Positionierungsmittel werden vorteilhaft gewährleistet, wenn der Verbindungsabschnitt mittig im Zentrierstift, d.h. beidseitig vom Befestigungsabschnitt umgeben, angeordnet wird. Somit bestimmt die Formgebung des Befestigungsabschnitts unabhängig von der Formgebung und der Verformung des Verbindungsabschnitts die im auswechselbaren Positionierungsmittel erforderliche Zentrieraufnahme zur Zentrierung.

In aller Regel ist davon auszugehen, dass es insbesondere aus ergonomischen Gründen, d.h. zur Gewährleistung eines möglichst hohen Tragekomforts, nicht möglich ist, das rechte und das linke auswechselbare Positionierungsmittel als Gleichteil auszuführen. Dennoch kann unter Berücksichtigung der Stellung des Zentrierstifts eine derartige Lösung geschaffen werden, wobei zwei verschiedene Positionierungsvarianten denkbar sind.

In einer ersten Lösung wird das als Gleichteil ausgeführte Positionierungsmittel in übereinstimmender Ausrichtung sowohl für die rechte als auch die linke Seite verwendet. In diesem Fall ist es einerseits erforderlich, dass eine durch die Zentrieraufnahme verlaufende Mittelebene senkrecht zur Auflagefläche im Bereich des auswechselbaren Positionierungsmittels ausgeführt ist, wobei es einer übereinstimmenden über die Mittelebene der Okklusionsvorrichtung symmetrischen Ausführung und Positionierung des Zentrierstifts sowie der Anlagefläche im Bereich des Positionierungsmittels bedarf. Anderseits ist es erforderlich, dass die beiden in Querrichtung betrachteten Außenflächen des auswechselbaren Positionierungsmittels symmetrisch über dessen Mittelebene ausgeführt werden müssen, so dass bei beidseitiger Verwendung die Zentrierungsfunktion der Miniplastschienen zueinander in Querrichtung gewährleistet werden kann. Die hierzu mögliche bzw. erforderliche Ausführung und Positionierung der Rastnase und der Rastvertiefung ergibt sich in naheliegender Weise.

In einer zweiten Lösung wird das als Gleichteil ausgeführte auswechselbare Positionierungsmittel rotationssymmetrisch um eine Mittelachse ausgeführt. Hierbei wird das auswechselbare Positionierungsmittel in um 180° gedrehter Stellung auf linker und rechter Seite eingesetzt. In diesem Fall ist es einerseits erforderlich, dass eine vordere Positionierungsfläche rotationssymmetrisch zu einer hinteren Positionierungsfläche am auswechselbaren Positionierungsmittel zur Festlegung der Position der Miniplastschienen in Längsrichtung ausgeführt ist. Weiterhin ist zur um 180° gedrehten Anbringung des auswechselbaren Positionierungsmittels zu beachten, dass der Zentrierstift in die Hochrichtung ausgerichtet wird oder zumindest bei einer über die Mittelebene der Okklusionsvorrichtung symmetrischen Abweichung nur in die Querrichtung geneigt wird und nicht in die Längsrichtung. D.h. entweder wird der Zentrierstift in Hochrichtung ausgerichtet oder weicht auf rechter und auf linker Seite um den gleichen Winkel aus der Hochrichtung in die Querrichtung ab. Die hierzu mögliche bzw. erforderliche Ausführung und Positionierung der Rastnase und der Rastvertiefung ergibt sich ebenso in naheliegender Weise.

Wenngleich es ebenso wie bei der bekannten Ausführungsform möglich ist, das auswechselbare Positionierungsmittel von Hand vom Zentrierstift abnehmen zu können, hat es sich als besonders vorteilhaft erwiesen, wenn zu diesem Zweck ein Demontagewerkzeug zur Verfügung gestellt wird. Dieses Demontagewerkzeug weist hierzu einen Demontagestift auf, welcher in eine Demontageausnehmung am auswechselbaren Positionierungsmittel eingesteckt werden kann. Mit Einstecken des Demontagestifts in die Demontageausnehmung am auswechselbaren Positionierungsmittel kann nunmehr das auswechselbare Positionierungsmittel mittels des Demontagewerkzeugs abgezogen bzw. abgeschoben werden. Somit erübrigt sich die ansonsten übliche Abnahme des auswechselbaren Positionierungsmittels durch ein Ergreifen desselben zwischen zwei Fingern, wobei zur Abnahme hohe Klemmkräfte von den Fingern aufgebracht werden mussten. Zur einfachen Handhabung ist es besonders vorteilhaft, wenn die Demontageausnehmung durchgehend ausgeführt ist und somit ein Einstecken des Demontagestifts in die Demontageausnehmung von beiden Seiten am auswechselbaren Positionierungsmittel möglich ist. Wesentlicher Vorteil der durchgehenden Demontageausnehmung ist jedoch die Verhinderung einer Einlagerung von schwer zu entfernenden Schmutzpartikeln.

Bei Realisierung einer Demontageausnehmung ist es weiterhin vorteilhaft, wenn sich diese quer zur Zentrieraufnahme im Wesentlichen in Querrichtung erstreckt. Die Anordnung der Demotageausnehmung unmittelbar an der Zentrieraufnahme ermöglicht weiterhin, dass bei Einstecken des Demontagestifts in dessen Folge unmittelbar ein Ausrasten der Rastnase aus der Rastvertiefung bewirkt wird. Entsprechend führt ein Einstecken des Demontagestifts in die Demontageausnehmung zu einer Verformung des elastischen Verbindungsabschnitts am Zentrierstift.

Hierbei ist es besonders vorteilhaft, wenn sich die Demontageausnehmung mit der Zentrieraufnahme im Bereich der Rastvertiefung überschneidet. Weiterhin ist es besonders vorteilhaft, wenn die Rastvertiefung unmittelbar von einem Abschnitt der Demontageausnehmung gebildet wird. Hierbei kann ein eingesteckter Demontagestift unmittelbar an der Rastnase zur Anlage gebracht werden und ebenso bei geringen Kräften ein Ausrasten der Rastnase aus der Rastvertiefung bewirken, so dass im Folgenden ein leichtes Abnehmen des auswechselbaren Positionierungsmittels von der Miniplastschiene ermöglicht wird.

Das Einstecken des Demontagestifts in die Demontageausnehmung zur Verformung des elastischen Verbindungsabschnitts wird vorteilhaft erleichtert, wenn der Demontagestift einen sich zum freien Ende verjüngenden Querschnitt aufweist, so dass das Einschieben des Demontagestifts quasi entlang einer schrägen Ebene zu einer Verformung des elastischen Verbindungsabschnitts führt.

Die Demontage des auswechselbaren Positionierungsmittels unter Einsatz des Demontagewerkzeugs wird weiterhin dadurch besonders verbessert, dass das Demontagewerkzeug in drehender Weise zur Demontage des auswechselbaren Positionierungsmittels eingesetzt werden kann. Hierbei erfolgt das Drehen des Demontagewerkzeugs um die Mittelachse des eingesteckten Demontagestifts, wobei eine Gleitfläche des Demontagewerkzeugs an der Anlagefläche der Miniplastschiene zur Anlage kommt. Beim Drehen des Demontagewerkzeugs um die Mittelachse des Demontagestifts gleitet die Gleitfläche entlang der Anlagefläche und bewirkt ein Abschieben des auswechselbaren Positionierungsmittels vom Zentrierstift.

Wenngleich die vorherige bekannte Ausführungsform sowie diese erfindungsgemäße Ausführungsform durch Einsatz von auswechselbaren Positionierungsmitteln eine schnelle geeignete Positionierung der mandibularen Miniplastschiene relativ zur maxillaren Miniplastschiene ermöglicht, ist es dennoch von Vorteil, wenn eine weitere Justierung, insbesondere in Längsrichtung, möglich ist. Hierzu wird die andere Miniplastschiene (ohne Zentrierstift) mit in Längsrichtung justierbaren Positionierungsmitteln versehen. Diese sind hier entsprechend auf der linken und rechten Seite anzuordnen und bewirken zusammen mit den auswechselbaren Positionierungsmitteln die Positionierung der Miniplastschienen zueinander in Längsrichtung. Entsprechend sind die justierbaren Positionierungsmittel nicht wie bei der Ausgangsform der Okklusionsschienenanordnung starr von der Miniplastschiene gebildet, sondern verstellbar angeordnet. Die Verstellung beschränkt sich hierbei lediglich auf eine Einstellung von deren Lage bei zugehöriger Miniplastschiene im Wesentlichen in der Längsrichtung. Zur Justierung wird in besonders vorteilhafter Weise eine Einstellschraube zwischen justierbarem Positionierungsmittel und zugehöriger Miniplastschiene vorgesehen.

Dabei ist es weiterhin besonders vorteilhaft, wenn bei Vorhandensein eines Demontagewerkzeugs dieses einen zusätzlichen Einstellstift zur Verstellung der Einstellschraube aufweist. Somit kann mit dem ohnehin vorhandenen Demontagewerkzeug zur Abnahme des auswechselbaren Positionierungsmittels ebenso eine Justierung des justierbaren Positionierungsmittels vorgenommen werden. Somit kann beispielsweise eine relative Änderung der Lage der Miniplastschienen zueinander in Längsrichtung in Millimeter-Sprüngen anhand des Auswechselns von den auswechselbaren Positionierungsmitteln vorgenommen werden, während hingegen eine Einstellung innerhalb eines Millimeters durch Justierung des justierbaren Positionierungsmittels vorgenommen wird.

Die Verwendung von auswechselbaren Positionierungsmitteln führt sowohl in bekannter Ausführungsform als auch in der erfindungsgemäßen Ausführungsform zu der Problematik einer möglichen Verwechslung der auswechselbaren Positionierungsmittel zur Anwendung an der linken Seite der Miniplastschiene sowie an der rechten Seite der Miniplastschiene, wenngleich der Fehler nach Aufstecken auf dem Zentrierstift unmittelbar offensichtlich ist. Daher ist es vorteilhaft, wenn der Verwender einen Hinweis erhält, welches auswechselbare Positionierungsmittel auf welchen der beiden Zentrierstifte auf der linken Seite bzw. auf der rechten Seite zu montieren ist. Diesbezüglich haben sich zwei verschiedene Gestaltungsformen zur Kennzeichnung als vorteilhaft herausgestellt.

In einer ersten Ausführungsform werden in vorteilhafter Weise die auswechselbaren Positionierungsmittel der linken Seite und der rechten Seite mit zwei verschiedenen Symbolen versehen, wobei diese in naheliegender Weise vorteilhaft mit dem Zeichen "L" und "R" versehen sind. Somit kann der Verwender der auswechselbaren Positionierungsmittel unmittelbar feststellen, an welcher Seite der Miniplastschiene die jeweiligen Positionierungsmittel zu montieren sind.

Um eine verbleibende Restunsicherheit bei in der Hand gehaltener Miniplastschiene auszuschließen, werden die Zentrierstifte auf der linken Seite und auf der rechten Seite ebenso in vorteilhafter Weise mit zwei unterschiedlichen Symbolen versehen, wobei die Miniplastschiene zumindest im Bereich des Zentrierstifts transluzent ist. Hierdurch sind die Symbole bei einer Anordnung im Sockelbereich des in der Miniplastschiene eingegossenen Zentrierstifts unmittelbar sichtbar, so dass eine Zuordnung zu den zu montierenden Positionierungsmitteln möglich wird. Naheliegend ist, dass es vorteilhaft ist, als Symbole die Zeichen "L" und "R" zu verwenden, wobei weiterhin in vorteilhafter Weise die Zentrierstifte und die auswechselbaren Positionierungsmittel mit übereinstimmenden Symbolen ausgeführt sind.

Weitgehend gleichwertig ist eine Anbringung der entsprechenden Symbole auf den justierbaren Positionierungsmitteln - sofern vorhanden -, wobei zu berücksichtigen gilt, dass bei der Montage der auswechselbaren Positionierungsmittel beide Miniplastschienen räumlich nah beieinander in zutreffender Stellung zueinander zu halten sind.

Möglich ist auch die Anbringung der Symbole unmittelbar auf der Miniplastschiene beispielsweise unterhalb der zu montierenden Positionierungsmittel. Jedoch ist in diesen Fällen der mögliche Mehraufwand bei der Werkzeugherstellung bzw. der Formgebung der Miniplastschienen zu berücksichtigen.

Die maßgebliche Funktion der auswechselbaren Positionierungsmittel ist es, eine Veränderung der Lage der mandibularen Miniplastschiene relativ zur maxillaren Miniplastschiene in Längsrichtung zu ermöglichen, wozu verschiedenartig auswechselbare Positionierungsmittel bereitgehalten werden, die jeweils eine unterschiedliche Lage der Zentrieraufnahme aufweisen. Zur Vermeidung einer Verwechslung der verschiedenartigen auswechselbaren Positionierungsmittel ist es daher weiterhin vorteilhaft, wenn die auswechselbaren Positionierungsmittel mit Symbolen versehen sind, die einerseits bei Zusammengehörigkeit, dem rechten auswechselbaren mit dem zugehörigen linken auswechselbaren Positionierungsmittel, mit übereinstimmender Lage der Zentrieraufnahme übereinstimmende Symbole aufweisen, jedoch unterschiedliche auswechselbare Positionierungsmittel für die linke Seite gleichfalls wie bei unterschiedlichen auswechselbaren Positionierungsmitteln für die rechte Seite mit jeweils unterschiedlichen Positionen der Zentrieraufnahme mit unterschiedlichen Symbolen versehen sind. In einfacher Weise können als Symbole Zahlen verwendet werden. Somit kann beispielsweise die Zahl 1 die erste mögliche Position eines auswechselbaren Positionierungsmittels darstellen, die Zahl 2 die nächstmögliche Stellung der zur Verfügung stehenden auswechselbaren Positionierungsmittel, usw..

Anstelle oder aber auch ergänzend zur Verwendung eines Symbols ist es zur Kennzeichnung der Zuordnung der auswechselbaren Positionierungsmittel vorteilhaft, wenn eine Farbkennzeichnung verwendet wird. Hierzu wird vorteilhafterweise das linke auswechselbare Positionierungsmittel und das rechte auswechselbare Positionierungsmittel zumindest abschnittsweise in einer unterschiedlichen Farbgebung ausgeführt.

Im Folgenden ist es weiterhin vorteilhaft, wenn die justierbaren Positionierungsmittel der linken Seite und der rechten Seite zumindest abschnittsweise eine mit dem auswechselbaren Positionierungsmittel übereinstimmende Farbgebung aufweisen. Somit kann der Verwender der Okklusionsschienenanordnung bei Auswahl der zu montierenden auswechselbaren Positionierungsmittel unmittelbar feststellen, an welcher Seite, d.h. der linken Seite oder der rechten Seite, das jeweilige Positionierungsmittel zu montieren ist.

Bei einer abschnittsweisen Farbgebung ist es besonders vorteilhaft, wenn sich die übereinstimmende Farbgebung auf der jeweiligen Außenseite am justierbaren Positionierungsmittel sowie auf der jeweiligen Außenseite am montierten auswechselbaren Positionierungsmittel befindet, so dass bei montiertem Positionierungsmittel die Farbgebung am auswechselbaren Positionierungsmittel und am justierbaren Positionierungsmittel nebeneinanderliegend überstimmend ist.

Zur Unterscheidung von auswechselbaren Positionierungsmitteln für die linke Seite und für die rechte Seite mit übereinstimmender Position der Zentrieraufnahme im Gegensatz zur Unterscheidung von nicht zusammengehörigen auswechselbaren Positionierungsmitteln der linken Seite sowie nicht zusammengehörigen auswechselbaren Positionierungsmitteln der rechten Seite ist es weiterhin vorteilhaft, wenn die zusammengehörigen Positionierungsmittel abschnittsweise eine übereinstimmende Farbgebung aufweisen, während hingegen nicht zusammengehörende Positionierungsmittel eine abweichende Farbgebung aufweisen. Somit wird nach Montage der Positionierungsmittel bei vorteilhaft auf der Innenseite liegender übereinstimmender Farbgebung vom rechten auswechselbaren Positionierungsmittel und linken auswechselbaren Positionierungsmittel eine korrekte Zusammengehörigkeit erkannt, während hingegen bei Farbunterschieden ein Fehler vorliegen muss.

Zu weiteren vorteilhaften Merkmalen der erfindungsgemäßen Ausführungsform wird diesbezüglich ausdrücklich auf die eingangs genannte Druckschrift WO 2013/143511 A1 Bezug genommen, deren vorteilhaften Ausführungsformen uneingeschränkt auch für die vorliegende Erfindung Geltung haben.

In den folgenden Figuren wird eine beispielhafte Okklusionsschienenanordnung mit Rastfunktion skizziert.

Es zeigen:
- Fig. 1: ein exemplarisches Ausführungsbeispiel einer erfindungsgemäßen Okklusionsschienenanordnung in perspektivischer Ansicht;
- Fig. 2: die Okklusionsschienenanordnung in Seitenansicht;
- Fig. 3: einen Schnitt durch die Okklusionsschienenanordnung;
- Fig. 4: die maxillare Miniplastschiene zur Ausführung aus Fig. 1;
- Fig. 5: die maxillare Miniplastschiene in Seitenansicht;
- Fig. 6: die mandibulare Miniplastschiene zur Ausführung aus Fig. 1 mit auswechselbaren Positionierungsmitteln;
- Fig. 7: die mandibulare Miniplastschiene in Seitenansicht;
- Fig. 8: den Zentrierstift der mandibulare Miniplastschiene mit auswechselbarem Positionierungsmittel;
- Fig. 9: den Zentrierstift der mandibulare Miniplastschiene;
- Fig. 10: verschiedene Ausführungen von auswechselbaren Positionierungsmitteln;
- Fig. 11: ein Demontagewerkzeug zur Demontage des auswechselbaren Positionierungsmittels und zur Einstellung des justierbaren Positionierungsmittels;
- Fig. 12: eine alternative Ausführungsform einer Miniplastschiene mit auswechselbaren Positionierungsmitteln;
- Fig. 13: die mandibulare Miniplastschiene in Seitenansicht;
- Fig. 14: ein Schnitt durch die mandibulare Miniplastschiene mit auswechselbaren Positionierungsmitteln.

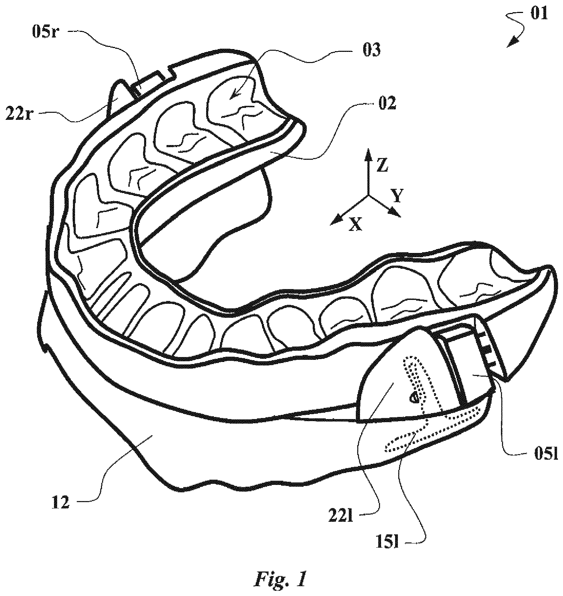

In der **Figur 1** wird eine beispielhafte Okklusionsschienenanordnung skizziert. Zu erkennen ist der Aufbau mit der maxillaren Miniplastschiene 02 sowie auf der unteren Seite mit der mandibularen Miniplastschiene 12. Hierbei weist die maxillare Miniplastschiene 02 eine entsprechende Zahnaufnahme 03 zur Anordnung auf der maxillaren Zahnreihe auf. Analog hierzu weist entsprechend die mandibulare Miniplastschiene 12 eine Zahnaufnahme 13 (unterhalb verdeckt liegend) zur Anordnung der mandibularen Miniplastschiene 12 auf der mandibularen Zahnreihe auf. Jede dieser Miniplastschienen 02, 12 weist hierbei beidseitig im molaren Bereich ein Positionierungsmittel 05 bzw. 22 auf. Das maxillare Positionierungsmittel 05r auf der rechten Seite sowie 051 auf der linken Seite ist justierbar - jedoch nicht ohne Weiteres auswechselbar - an der maxillaren Miniplastschiene 02 befestigt. Hingegen wird das mandibulare Positionierungsmittel 22 als auswechselbares Positionierungsmittel 22r für die rechte Seite sowie 221 für die linke Seite ausgeführt. Diese sind jeweils auf Montagemitteln in Form von Zentrierstiften 15r und 151 angebracht.

Die auswechselbaren Positionierungsmittel 22 der mandibularen Miniplastschiene 12 weisen weiterhin nach innen weisende Führungsflächen 24 auf, wohingegen die hierzu komplementären Positionierungsmittel der maxillaren Miniplastschiene 02 von deren Außenseiten gleichfalls als Führungsflächen 07 gebildet werden. Diese 07, 24 dienen hierbei der Zentrierung und Führung der Miniplastschienen 02, 12 zueinander in der Querrichtung (Y). Die Positionierung in der Längsrichtung (X) wird mittels der Positionierungsflächen 06 an den justierbaren Positionierungsmitteln 05 sowie gegenüberliegend zusammenwirkend mit den Positionierungsflächen 23 bei dem auswechselbaren Positionierungsmittel 22 realisiert.

Schematisch skizziert wird die erfindungsgemäße Ausführung des Zentrierstifts 15 mit einem elastisch verformbaren Verbindungsabschnitt 18, an dessen Ende sich die Rastnase 19 befindet. Diese verrastet in einer Rastvertiefung 26, welche wiederum von der Demontageausnehmung 27 gebildet wird, welche das auswechselbare Positionierungsmittel 22 in Querrichtung durchdringt.

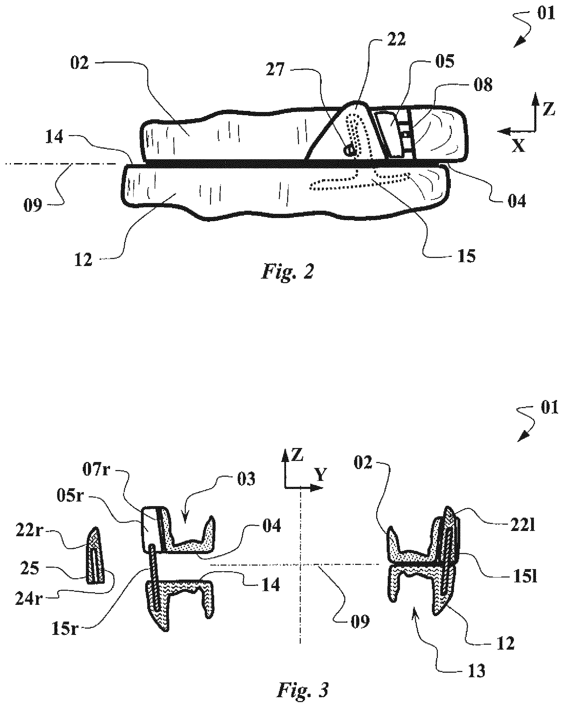

In der **Figur 2** wird die Okklusionsschienenanordnung 01 aus Fig. 1 in der Seitenansicht skizziert. Zu erkennen ist die Anordnung der Anlageflächen 04 der maxillaren Miniplastschiene 02 gegenüberliegend der Anlagefläche 14 der mandibularen Miniplastschiene 12, welche im Wesentlichen bei geschlossenem Gebiss in der Okklusionsebene 09 liegen. Hierbei ist des Weiteren zu erkennen, dass sich das maxillare Positionierungsmittel 05 der maxillaren Miniplastschiene 02 vollständig oberhalb der Anlagefläche 04 befindet, während hingegen das mandibulare Positionierungsmittel 22 oberhalb der entsprechenden Anlagefläche 14 auf der mandibularen Miniplastschiene 12 angeordnet ist. Weiterhin erkennbar ist die Gestaltung der Positionierungsflächen 06, 23, welche zunächst einmal eben gestaltet sind, jedoch gegenüber der Hochrichtung (Z) nach vorne geneigt ausgeführt sind. Hierbei ist des Weiteren (vgl. Fig. 1) zu berücksichtigen, dass das mandibulare auswechselbare Positionierungsmittel 22 sich außerhalb und vor dem maxillaren justierbaren Positionierungsmittel 05 befindet. Somit wird sichergestellt, dass beim Schließen des Gebisses ein Vorziehen der mandibularen Miniplastschiene 12 und somit des Unterkiefers zur wirksamen Schlafapnoe-Therapie erfolgt.

Die Einstellbarkeit des justierbaren Positionierungsmittels 05 wird ermöglicht durch dessen in Längsrichtung verschiebbare Lagerung an der maxillaren Miniplastschiene 02, wobei zur Justierung eine Einstellschraube 08 vorgesehen ist.

Die **Figur 3** skizziert ergänzend einen Schnitt durch die Okklusionsschienenanordnung 01, wobei diese auf der linken Seite als Explosionsdarstellung skizziert ist und rechter Hand in aufeinanderliegender Stellung. Zu erkennen ist wiederum die Anordnung der maxillaren Miniplastschiene 02 mit der maxillaren Zahnaufnahme 03 sowie gegenüberliegend der mandibularen Miniplastschiene 12 mit der entsprechenden Zahnaufnahme 13. Diese Miniplastschienen 02, 12 weisen jeweils entsprechende Anlageflächen 04, 14 auf, welche bei geschlossenem Gebiss in der Okklusionsebene 09 zur Anlage kommen. Die relative Führung in Querrichtung (Y) wird mittels der Führungsflächen 07, gebildet von der Außenfläche der maxillaren Miniplastschiene 02, in Kontakt mit den Führungsflächen 24, gebildet von der Innenseite des auswechselbaren Positionierungsmittels 22, sichergestellt. Diese Führungsflächen 07, 24 sind hierbei gegenüber der Hochrichtung (Z) nach außen um einen Neigungswinkel 19 geneigt. Somit wird sichergestellt, dass beim Schließen des Gebisses die Führungsflächen 07, 24 zueinander finden, ohne dass es zu einem Aufstoßen des mandibularen Positionierungsmittels 22 an der Anlagefläche 04 der maxillaren Miniplastschiene 02 kommt.

Weiterhin zu erkennen ist die Befestigungsweise des auswechselbaren Positionierungsmittels 22 an der mandibularen Miniplastschiene 12 durch Aufsetzen der Zentrieraufnahme 25 auf den Zentrierstift 15.

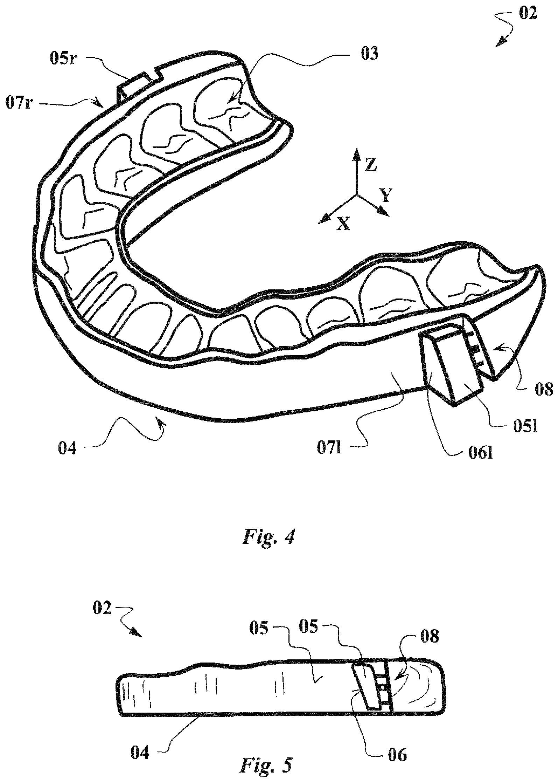

In der **Figur 4** wird hierzu nochmals die maxillare Miniplastschiene 02 skizziert. Zu erkennen ist wiederum die Anordnung der Zahnaufnahme 03 zur Anbringung der maxillaren Miniplastschiene 02 auf der maxillaren Zahnreihe des Patienten. Weiterhin sind die Positionierungsmittel 05r und 051 im molaren Bereich mit den Positionierungsflächen 06 zu erkennen. Die Positionierungsmittel zur Führung in Querrichtung werden von der Außenseite der maxillaren Miniplastschiene 02 mit den Führungsflächen 071 und 07r realisiert. Unterseitig (verdeckt liegend) befindet sich die Anlagefläche 04, welche hier eine ebene Form aufweist und zur Anlage an der jeweils anderen Miniplastschiene 12 bestimmt ist.

In der **Figur 5** wird ergänzend zu Fig. 4 nochmals die maxillare Miniplastschiene 02 in der Seitenansicht skizziert, mit der in Querrichtung (Y) und Hochrichtung (Z) ausgerichteten Positionierungsfläche 06 des maxillaren Positionierungsmittels 05 sowie der in Längsrichtung (X) und Hochrichtung (Z) ausgerichteten Führungsfläche 07.

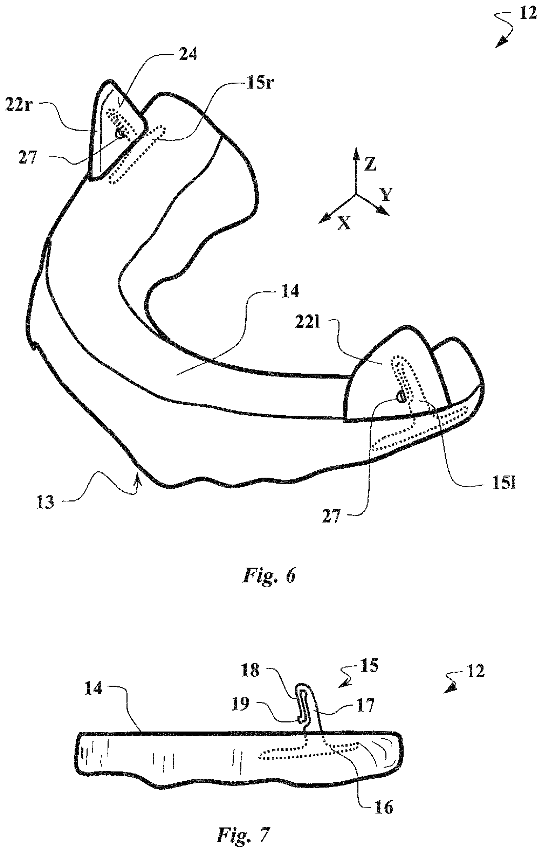

Die **Figur 6** skizziert die mandibulare Miniplastschiene 12 aus Fig. 1, bei welcher sich (wie bei Fig. 1) die Zahnaufnahme 13 untenliegend (verdeckt) befindet. Die dargestellte Oberseite bildet die Anlagefläche 14, welche gleichfalls eben ausgeführt ist und zur Anlage an der maxillaren Anlagefläche 04 der maxillaren Miniplastschiene 02 bestimmt ist.

Die relative Stellung zwischen den Miniplastschienen 02, 12 wird hier mittels auswechselbarer Positionierungsmittel 22r, 221 realisiert, welche jeweils eine zum maximalen Positionierungsmittel 05 komplementäre Führungsfläche 24 sowie eine entsprechende komplementäre Positionierungsfläche 23 aufweisen. Zu erkennen ist weiterhin die skizzierte Befestigungsweise der Positionierungsmittel 22 auf der mandibularen Miniplastschiene 12, welche mittels im Grundkörper der Miniplastschiene verankerter Zentrierstifte 151, 15r erfolgt. Hierbei stellt sich das auswechselbare Positionierungsmittel 22 in Art einer Finne dar, bei der die zur Mitte hin weisende Fläche die Führungsfläche 24 und die nach hinten liegende Vorderkante der Finne die Positionierungsfläche 23 bildet.

Die **Figur 7** zeigt ergänzend zu Fig. 6 die mandibulare Miniplastschiene 12 in der Seitenansicht, jedoch ohne deren zugehörige Positionierungsmittel 22. Zu erkennen ist der Zentrierstift 15, welcher über der Anlagefläche 14 hervorsteht und hierbei mit einem Sockelabschnitt 16 in der Miniplastschiene 12 verankert ist.

Im Gegensatz zu der bekannten Ausführungsform weist dieser Zentrierstift 15 einen oberhalb der Anlagefläche 14 hervorstehenden Befestigungsabschnitt 17 auf, an den sich am oberen freien Ende des Zentrierstifts 15 der Verbindungsabschnitt 18 anschließt. Dieser erstreckt sich um ein freies Auge im Halbkreis und verläuft anschließend in Richtung zur Anlagefläche 14 näherungsweise parallel zum Befestigungsabschnitt 17. Am freien Ende des Verbindungsabschnitts 18 befindet sich die erhabene Rastnase 19.

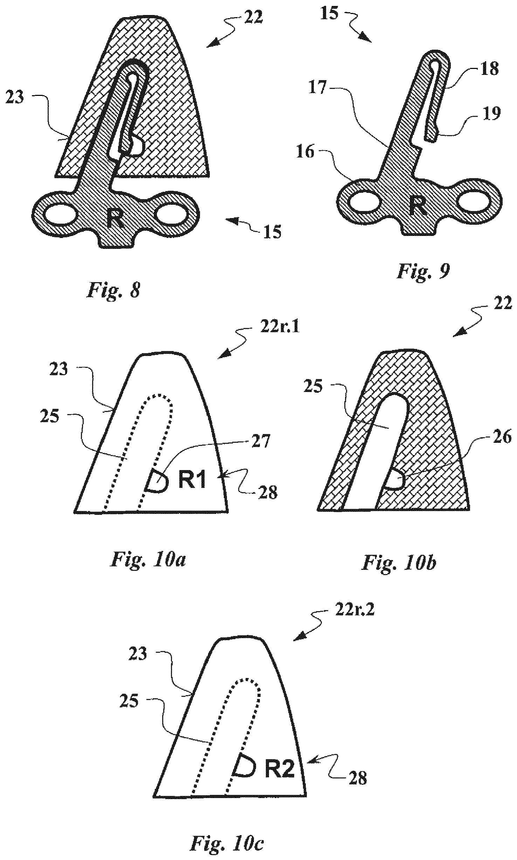

In der **Figur 8** wird der Zentrierstift 15 mit montiertem auswechselbarem Positionierungsmittel 22 in einem Längsschnitt skizziert. Zur weiteren Erläuterung wird hinsichtlich des Zentrierstifts 15 auf die **Figur 9** verwiesen, wobei das auswechselbare Positionierungsmittel 22 im Schnitt in der **Figur 10b** separat dargestellt ist.

Zu erkennen ist zunächst einmal der Aufbau des Zentrierstifts 15 mit dem in der Miniplastschiene 12 verankerten Sockelabschnitt 16. Erhaben aus der Anlagefläche 14 erstreckt sich der starr angeordnete Befestigungsabschnitt 17, welcher beim Fügen des auswechselbaren Positionierungsmittels 22 im Wesentlichen nicht verformt wird. Am von der Anlagefläche 14 fernen Ende des Befestigungsabschnitts 17 schließt sich der elastisch verformbare Verbindungsabschnitt 18 an, wobei sich dieser zunächst einmal um ein freies Auge um einen Halbkreis mit circa 175° erstreckt. Darauf folgend erstreckt sich der elastisch verformbare Verbindungsabschnitt 18 in Richtung zur Anlagefläche 14 näherungsweise parallel zum Befestigungsabschnitt 17. Am freien Ende des Verbindungsabschnitts 18 ist die Rastnase 19 angeordnet, welche in der Rastvertiefung 26 der Zentrieraufnahme 25 eintauchen kann.

Bei nicht montiertem Positionierungsmittel 22 weitet sich der Zentrierstift 15 ausgehend von seinem freien Ende in Richtung zur Anlagefläche 14 geringfügig auf, während demgegenüber bei montiertem Positionierungsmittel 22 der Zentrierstift 15 mit einer Vorderkante am Befestigungsabschnitt 17 im Wesentlichen parallel zu einer hinteren Kante am Verbindungsabschnitt 18 geformt ist. Zum Ermöglichen der elastischen Verformung des Verbindungsabschnitts 18 weitet sich ein vom freien Auge ausgehender Spalt zum Ende des Verbindungsabschnitts 18 hin auf. Somit ist uneingeschränkt die Verformbarkeit des Verbindungsabschnitts 18 beim Eintauchen desselben in die Zentrieraufnahme 25 zur Realisierung der Rastfunktion gewährleistet. Weiterhin ist den Figuren zu entnehmen, dass die Zentrieraufnahme 25 im Wesentlichen parallel zu einer vorderen Positionierungsfläche 23 am auswechselbaren Positionierungsmittel 22 verläuft und hierbei einen im Wesentlichen konstanten Querschnitt aufweist. Entsprechend der Neigung der Positionierungsfläche 23 am Positionierungsmittel 22 ist die Zentrieraufnahme 25 und somit der Zentrierstift 15 leicht gegenüber der Hochachse (Z) geneigt.

In dieser vorteilhaften Ausführung wird die Rastvertiefung 26 der Zentrieraufnahme 25 von einem Abschnitt einer durchgehenden Demontageausnehmung 27 gebildet, wie sich dies aus der gemeinsamen Betrachtung der Figuren 10a und 10b ergibt.

Aus der Gegenüberstellung der Figuren 10a und 10c werden die verschiedenen Stellungen der Positionierungsmittel 22 an der Miniplastschiene 12 in Längsrichtung (X) offensichtlich. Hierzu zeigen die beiden exemplarisch dargestellten auswechselbaren Positionierungsmittel 22r.1 und 22r.2 die Anordnung der Zentrieraufnahme 25 an jeweils unterschiedlicher Stelle relativ zur Positionierungsfläche 23.

Weiterhin schematisch dargestellt ist in den **Figuren 8** bis **10** die Kennzeichnung von Zentrierstift 15 und Positionierungsmittel 22 anhand von übereinstimmenden Symbolen 28, in diesem Fall mit dem Zeichen "R" für die Verwendung an der rechten Seite der Miniplastschiene 12. Weiterhin sind ergänzend unterschiedliche Symbole 28 bei den unterschiedlichen Ausführungsformen in Fig. 10a und 10b angebracht, wobei exemplarisch das Zeichen "1" für ein erstes Positionierungsmittel 22r.1 und das Zeichen "2" für ein zweites Positionierungsmittel 22r.2 in einer Reihe von unterschiedlichen Positionierungsmitteln 22 verwendet wird.

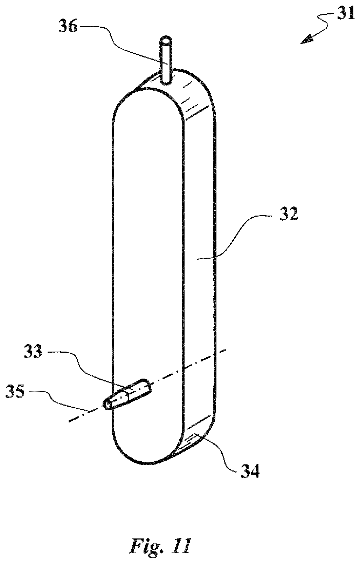

In der **Figur 11** wird ein Beispiel für ein Demontagewerkzeug 31 skizziert, mit dessen Hilfe in besonders vorteilhafter Weise eine Demontage des auswechselbaren Positionierungsmittels 22 sowie eine vorteilhafte Einstellung des justierbaren Positionierungsmittels 05 möglich ist. Hierzu weist das Demontagewerkzeug 31 an einem Griff 32 einen im Wesentlichen zylindrischen Demontagestift 33 auf, welcher sich zum freien Ende hin verjüngt. Der Demontagestift 33 bildet hierbei eine Mittelachse 35, um die das Demontagewerkzeug 31 bei dessen Handhabung am Griff 32 gedreht werden kann. Hierbei kommt beim Schwenken des Griffs 32 eine Gleitfläche 34 an der Anlagefläche 14 an der zum auswechselbaren Positionierungsmittel 22 zugehörigen Miniplastschiene 12 zur Anlage. Während des Drehens des Griffs 32 um die Mittelachse 35 erfolgt das Abschieben des auswechselbaren Positionierungsmittels 22 von dem Zentrierstift 15 der Miniplastschiene 12. Hierzu ist es naheliegend erforderlich, dass sich der Abstand der Gleitfläche 34 zur Mittelachse 35 während des Drehens um die Mittelachse 35 in Anlage an die Anlagefläche 14 vergrößert.

Bei gemeinsamer Betrachtung der **Figur 8** mit der **Figur 11** wird die weitere vorteilhafte Funktionsweise des Demontagewerkzeugs 31 ersichtlich. Beim Eintauchen des Demontagestifts 33 in die Demontageausnehmung 27 wird die in der Rastvertiefung 26 befindliche Rastnase 19 vom Demontagestift 33 verdrängt und somit ein Ausrasten bewirkt.

Das Demontagewerkzeug 31 weist in dieser vorteilhaften Ausführung weiterhin einen Einstellstift 36 auf, welcher zur Betätigung einer Einstellschraube 08 zur Justierung des justierbaren Positionierungsmittels 05 verwendet werden kann.

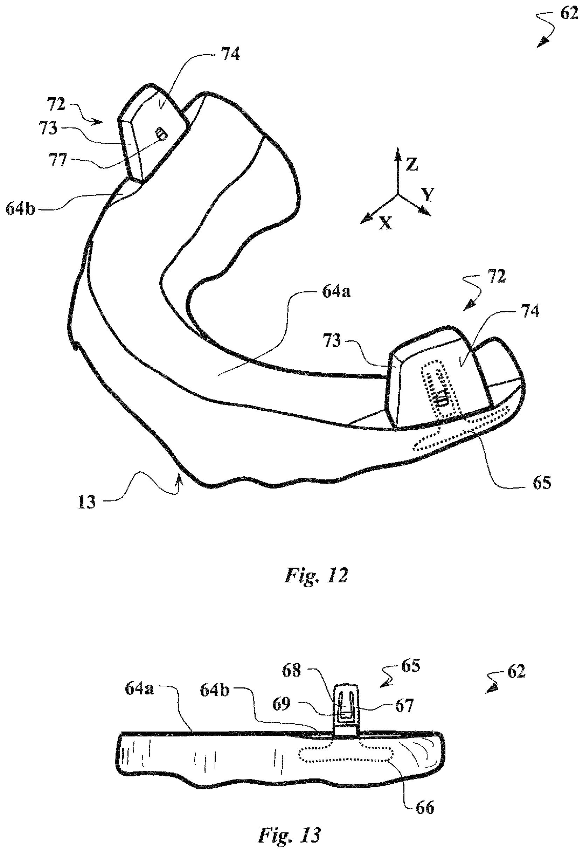

In der **Figur 12** wird eine alternative Ausführungsform für eine mandibulare Miniplastschiene 62 offenbart. Diese weist ebenso wie das vorherige Ausführungsbeispiel die auswechselbaren Positionierungsmittel 72 auf. Im Gegensatz zur vorherigen Ausführungsform ist jedoch nunmehr das auswechselbare Positionierungsmittel 72 rotationssymmetrisch ausgeführt, so dass es sowohl für die linke Seite als auch für die rechte Seite in identischer Ausführungsform zum Einsatz kommen kann. Entsprechend weist das auswechselbare Positionierungsmittel sowohl an der vorderen Kante eine Positionierungsfläche 73 als auch an der hinteren Kante eine Positionierungsfläche 73 auf. Ebenso weist dieses Ausführungsbeispiel auf der zur Innenseite weisenden Fläche als auch ebenso auf der nach außen weisenden Seite eine Führungsfläche 74 auf. Zur Realisierung einer rotationssymmetrischen Anbringung des auswechselbaren Positionierungsmittels an der mandibularen Miniplastschiene 62 ist die Anlagefläche 64 ausgehend von der überwiegend ebenen Anlagefläche 64a zur Auflage der maxillaren Miniplastschiene 02 im Bereich der auswechselbaren Positionierungsmittel 72 geringfügig als Anlagefläche 64b geneigt. Die Schrägstellung der geneigten Anlagefläche 64b im Gegensatz zur ebenen Anlagefläche 64a entspricht der Winkelabweichung des in der mandibularen Miniplastschiene 62 verankerten Zentrierstifts 65 gegenüber der Hochachse (Z). Ein weiterer Unterschied gegenüber dem vorherigen Ausführungsbeispiel zeigt die mandibulare Miniplastschiene 62 mit der geänderten Ausführungsform des Zentrierstifts 65.

Wie in **Figur 13** zu erkennen ist, wird der Zentrierstift 65 ebenso aus einem Sockelabschnitt 66 und einem sich daran oberhalb der Anlagefläche 64 anschließenden Befestigungsabschnitt 67 gebildet. Der sich am oberen Ende des Befestigungsabschnitts 67 anschließende Verbindungsabschnitt 68 weist ebenso in Richtung der Anlagefläche 64, wobei nunmehr jedoch der Verbindungsabschnitt 68 mittig im Zentrierstift 65 angeordnet ist und aus der Fläche des Befestigungsabschnitts 67 hervorsteht. Am unteren Ende des Verbindungsabschnitts 68 befindet sich wiederum die Rastnase 69 zur Fixierung des auswechselbaren Positionierungsmittels 72.

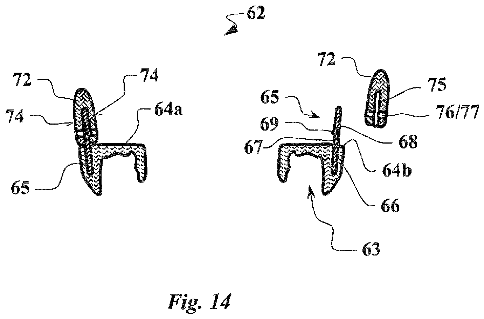

Hierzu skizziert die nachfolgende **Figur 14** nochmals im Querschnitt die mandibulare Miniplastschiene 62 mit dem auswechselbaren Positionierungsmittel 72, wobei nunmehr zu erkennen ist, dass der Zentrierstift 65 geringfügig gegenüber der Anlagefläche 64a, d.h. der Okklusionsebene, geneigt ist. Die entsprechende Neigung weist übereinstimmend die geneigte Anlagefläche 64b auf. Die Neigung der Anlagefläche 64b dient lediglich der Realisierung der beliebigen Positionierung eines rotationssymmetrischen auswechselbaren Positionierungsmittels 72, so dass dessen 72 Unterseite jeweils in beiden Stellungen flächig auf der Anlagefläche 64b aufliegt. In diesem besonderen Ausführungsbeispiel ist das auswechselbare Positionierungsmittel 72 dahingehend spezifiziert, dass dieses 72 sowohl auf der linken als auch auf der rechten Seite in beliebiger Ausrichtung, d.h. jeweils um 180° gedreht, auf dem Zentrierstift 65 angebracht werden kann. Hierzu sind im jeweiligen auswechselbaren Positionierungsmittel 72 beidseitig der mittigen Zentrieraufnahme 75 die erforderlichen Rastvertiefungen 76 angeordnet, welche 76 analog dem vorherigen Ausführungsbeispiel von der Demontageausnehmung 77 gebildet werden.

### Bezugszeichenliste

- 01: Okklusionsschienenanordnung
- 02: Maxillare Miniplastschiene
- 03: Zahnaufnahme
- 04: Anlagefläche
- 05: Justierbares Positionierungsmittel
- 06: Positionierungsfläche
- 07: Führungsfläche
- 08: Einstellschraube
- 09: Okklusionsebene

- 12, 62: Mandibulare Miniplastschiene
- 13, 63: Zahnaufnahme
- 14: Anlagefläche
- 64a: Anlagefläche für maxillare Miniplastschiene
- 64b: Anlagefläche für auswechselbares Positionierungsmittel
- 15, 65: Montagemittel / Zentrierstift
- 16, 66: Sockelabschnitt
- 17, 67: Befestigungsabschnitt
- 18, 68: Verbindungsabschnitt
- 19, 69: Rastnase

- 72: Rotationssymmetrisches auswechselbares Positionierungsmittel
- 221: Rechtes auswechselbares Positionierungsmittel
- 22r: Linkes auswechselbares Positionierungsmittel
- 23, 73: Positionierungsfläche
- 24, 74: Führungsfläche
- 25, 75: Zentrieraufnahme
- 26, 76: Rastvertiefung
- 27, 77: Demontageausnehmung

- 31: Demontagewerkzeug
- 32: Griff
- 33: Demontagestift
- 34: Gleitfläche
- 35: Mittelachse des Demontagestifts
- 36: Einstellstift

- X: Längsrichtung
- Y: Querrichtung
- Z: Hochrichtung

## Patentansprüche

1. Okklusionsschienenanordnung (01), insbesondere zur Schlafapnoe-Therapie, mit einer auf der maxillaren Zahnreihe anordenbaren maxillaren Miniplastschiene (02) und einer auf der mandibularen Zahnreihe anordenbaren mandibularen Miniplastschiene (12, 62), wobei die Miniplastschienen (02, 12, 62) mit ebenen Anlageflächen (04, 14, 64a) ausgeführt sind, die (04, 14, 64a) gegeneinander zur Anlage gebracht werden können, wobei in einer Miniplastschiene (12, 62) auf der linken Seite und auf der rechten Seite im molaren Bereich jeweils ein aus der Anlagefläche (14, 64b) herausstehender Zentrierstift (15, 65) verankert ist, wobei linke und rechte auswechselbare Positionierungsmittel (221, 22r, 72) eine zum jeweiligen Zentrierstift (15, 65) komplementäre Zentrieraufnahme (25, 75) aufweisen und an der Miniplastschiene (12, 62) mit Anlage auf der Anlagefläche (14, 64b) auswechselbar befestigt sind, wobei durch einen Formschluss zwischen den auswechselbaren Positionierungsmitteln (22, 72) und Positionierungsmitteln (05, 07) der anderen Miniplastschiene (02) die relative Stellung der Miniplastschienen (02, 12, 62) zueinander in Längsrichtung (X) und/oder in Querrichtung (Y) definierbar ist, **dadurch gekennzeichnet,**
**dass** der Zentrierstift (15, 65) eine Rastnase (19, 69) und die Zentrieraufnahme (25, 75) eine Rastvertiefung (26, 76) aufweist, wobei die Rastnase (19, 69) durch Einrasten in der Rastvertiefung (26, 76) eine Fixierung des auswechselbaren Positionierungsmittels (22, 72) an der Miniplastschiene (12, 62) bewirkt, wobei der Zentrierstift (15, 65) einen in die Miniplastschiene (12, 62) eingegossenen Sockelabschnitt (16, 66) und einen starr über der Anlagefläche (14, 64) überstehenden Befestigungsabschnitt (17, 67) aufweist, an dem (17, 67) ein elastisch verformbarer Verbindungsabschnitt (18, 68) angeformt ist, an dessen (18, 68) freiem Ende die Rastnase (19, 69) angeordnet ist.

2. Okklusionsschienenanordnung (01) nach Anspruch 1,
**dadurch gekennzeichnet,**
**dass** der Zentrierstift (15, 65) einstückig ausgeführt ist; und/oder dass der Zentrierstift (15, 65) aus einem Metallblech, insbesondere durch Laserschneiden, hergestellt ist.

3. Okklusionsschienenanordnung (01) nach Anspruch 1 oder 2,
**dadurch gekennzeichnet,**
**dass** sich der elastisch verformbare Verbindungsabschnitt (18, 68) vom von der Anlagefläche (14, 64) entfernten Ende des starren Befestigungsabschnitts (17, 67) ausgehend entlang des Befestigungsabschnitts (17, 67) in Richtung zur Anlagefläche (14, 64) erstreckt.

4. Okklusionsschienenanordnung (01) nach Anspruch 3,
**dadurch gekennzeichnet,**
**dass** sich der elastisch verformbare Verbindungsabschnitt (18) am starren Befestigungsabschnitts (17) anschließt und dort beginnend bogenförmig um ein freies Auge um 170° ± 10°, insbesondere um 175° ± 5°, verläuft.

5. Okklusionsschienenanordnung (01) nach Anspruch 4,
**dadurch gekennzeichnet,**
**dass** sich im nicht eingerasteten Zustand ein Spalt zwischen dem Befestigungsabschnitt (17) und dem Verbindungsabschnitt (18) ausgehend vom Ende des Befestigungsabschnitts (17), insbesondere ausgehend vom freien Auge, aufweitet.

6. Okklusionsschienenanordnung (01) nach einem der Ansprüche 3 bis 5,
**dadurch gekennzeichnet,**
**dass** im eingerasteten Zustand eine vordere Fläche am Befestigungsabschnitt (17) und eine hintere Fläche am Verbindungsabschnitt (18) im Wesentlichen parallel zueinander verlaufen.

7. Okklusionsschienenanordnung (01) nach Anspruch 3,
**dadurch gekennzeichnet,**
**dass** sich im nicht eingerasteten Zustand der Verbindungsabschnitt (68) gegenüber dem Befestigungsabschnitt (67) in der Querrichtung (Y) ausgehend vom Ende des Befestigungsabschnitts (67) zunehmend abhebt.

8. Okklusionsschienenanordnung (01) nach einem der Ansprüche 1 bis 7,
**dadurch gekennzeichnet,**
**dass** das auswechselbare Positionierungsmittel (22, 72) zumindest eine, insbesondere durchgehende, Demontageausnehmung (27, 77) aufweist, in die ein Demontagestift (33) eines Demontagewerkzeugs (31) zur Abnahme des auswechselbaren Positionierungsmittels (22, 72) von der Miniplastschiene (12, 62) eingesteckt werden kann, wobei die Demontageausnehmung (27, 77) quer zur Zentrieraufnahme (25, 75) ausgerichtet ist, wobei ein eingesteckter Demontagestift (33) ein Ausrasten der Rastnase (19, 69) aus der Rastvertiefung (26, 76) bewirkt.

9. Okklusionsschienenanordnung (01) nach Anspruch 8,
**dadurch gekennzeichnet,**
**dass** die Demontageausnehmung (27, 77) die Zentrieraufnahme (25, 75) im Bereich der Rastvertiefung (26, 76) schneidet, insbesondere dass die Rastvertiefung (26,76) von einem Abschnitt der Demontageausnehmung (27, 77) gebildet wird, wobei ein eingesteckter Demontagestift (33) an der Rastnase (19, 69) zur Anlage kommt und hierbei ein Ausrasten der Rastnase (19, 69) aus der Rastvertiefung (26, 76) bewirkt.

10. Okklusionsschienenanordnung (01) nach Anspruch 8 oder 9,
**dadurch gekennzeichnet,**
**dass** das freie Ende des Demontagestifts (33) einen sich verjüngenden Querschnitt aufweist.

11. Okklusionsschienenanordnung (01) nach einem der Ansprüche 8 bis 10,
**dadurch gekennzeichnet,**
**dass** das Demontagewerkzeug (31) nach Einstecken des Demontagestifts (33) in die Demontageausnehmung (27, 77) mit einer Gleitfläche (34) an den Anlageflächen (14, 64) der Miniplastschiene (12, 62) zur Anlage kommen kann, und wobei das auswechselbare Positionierungsmittel (22, 72) durch Drehen des Demontagewerkzeugs (31) um die Mittelachse (35) des eingesteckten Demontagestifts (33) vom Zentrierstift (15, 65) abgeschoben wird.

12. Okklusionsschienenanordnung (01) nach einem der Ansprüche 1 bis 11,
**dadurch gekennzeichnet,**
**dass** die andere Miniplastschiene (02) auf der linken Seite und auf der rechten Seite im Wesentlichen in Längsrichtung (X) justierbare Positionierungsmittel (05) aufweist.

13. Okklusionsschienenanordnung (01) nach einem der Ansprüche 8 bis 12,
**dadurch gekennzeichnet,**
**dass** die Justierung mittels einer Einstellschraube (08) vorgenommen werden kann, wobei am Demontagewerkzeug (31) ein zusätzlicher Einstellstift (36) zur Verstellung der Einstellschraube (08) angeordnet ist.

## Claims

1. An occlusal splint arrangement (01), in particular for sleep apnoea therapy, comprising a maxillary miniplast splint (02) that can be arranged on the maxillary row of teeth and a mandibular miniplast splint (12, 62) that can be arranged on the mandibular row of teeth, the miniplast splints (02, 12, 62) being formed with even contact surfaces (04, 14, 64a) which can be brought to bear against each other, wherein a centring pin (15, 65) which protrudes out of the contact surface (14, 64b) is anchored in one miniplast splint (12, 62) both on the left side and on the right side in the molar area, left and right exchangeable positioning means (221, 22r, 72) having a centring recess (25, 75) complementary to the respective centring pin (15, 65) and being exchangeably attached to the miniplast splint (12, 62), bearing against the contact surface (14, 64b), the relative position of the miniplast splints (02, 12, 62) towards each other being definable in the longitudinal direction (X) and/or in the transverse direction (Y) by way of a form fit between the exchangeable positioning means (22, 72) and positioning means (05, 07) of the other miniplast splint (02),
**characterised in that**
the centring pin (15, 65) has a latch lug (19, 69) and the centring recess (25, 75) has a latching depression (26, 76), the latch lug (19, 69) causing a fixation of the exchangeable positioning means (22, 72) on the miniplast splint (12, 62) by latching into the latching depression (26, 76), the centring pin (15, 65) having a base portion (16, 66) cast into the miniplast splint (12, 62) and a fastening portion (17, 67) rigidly protruding beyond the contact surface (14, 64), an elastically deformable connecting portion (18, 68) being moulded thereto at whose free end the latch lug (19, 69) is arranged.

2. The occlusal splint arrangement (01) according to claim 1,
**characterised in that**
the centring pin (15, 65) is formed as one piece; and/or the centring pin (15, 65) is produced from a metal sheet, in particular by laser cutting.

3. The occlusal splint arrangement (01) according to claim 1 or 2,
**characterised in that**
starting from the end of the rigid fastening portion (17, 67) that is distant from the contact surface (14, 64), the elastically deformable connecting portion (18, 68) extends along the fastening portion (17, 67) in the direction towards the contact surface (14, 64).

4. The occlusal splint arrangement (01) according to claim 3,
**characterised in that**
the elastically deformable connecting portion (18) is adjacent to the rigid fastening portion (17) and, beginning there, the connecting portion extends in a bent shape around a free eye by 170° ± 10°, in particular by 175° ± 5°.

5. The occlusal splint arrangement (01) according to claim 4,
**characterised in that**
in the unlatched state, a gap widens between the fastening portion (17) and the connecting portion (18) starting from the end of the fastening portion (17), in particular starting from the free eye.

6. The occlusal splint arrangement (01) according to any of the claims 3 to 5,
**characterised in that**
in the latched state, a front surface on the fastening portion (17) and a rear surface on the connecting portion (18) run substantially parallel to each other.

7. The occlusal splint arrangement (01) according to claim 3,
**characterised in that**
in the unlatched state, the connecting portion (68) increasingly rises with respect to the fastening portion (67) in the transverse direction (Y) starting from the end of the fastening portion (67).

8. The occlusal splint arrangement (01) according to any of the claims 1 to 7,
**characterised in that**
the exchangeable positioning means (22, 72) has at least one in particular continuous disassembly recess (27, 77) into which a disassembly pin (33) of a disassembling tool (31) can be inserted to remove the exchangeable positioning means (22, 72) from the miniplast splint (12, 62), the disassembly recess (27, 77) being oriented perpendicularly to the centring recess (25, 75), an inserted disassembly pin (33) causing the latch lug (19, 69) to latch out of the latching depression (26, 76).

9. The occlusal splint arrangement (01) according to claim 8,
**characterised in that**
the disassembly recess (27, 77) intersects the centring recess (25, 75) in the area of the latching depression (26, 76), in particular that the latching depression (26, 76) is formed by a portion of the disassembly recess (27, 77), an inserted disassembly pin (33) coming to bear against the latch lug (19, 69) and thereby causing the latch lug (19, 69) to latch out of the latching depression (26, 76).

10. The occlusal splint arrangement (01) according to claim 8 or 9,
**characterised in that**
the free end of the disassembly pin (33) has a tapering cross-section.

11. The occlusal splint arrangement (01) according to any of the claims 8 to 10,
**characterised in that**
after the disassembly pin (33) has been inserted into the disassembly recess (27,77), the disassembling tool (31) can come to bear with a sliding surface (34) against the contact surfaces (14, 64) of the miniplast splint (12, 62), the exchangeable positioning means (22, 72) being pushed off the centring pin (15, 65) by a rotation of the disassembling tool (31) about the centre axis (35) of the inserted disassembly pin (33).

12. The occlusal splint arrangement (01) according to any of the claims 1 to 11,
**characterised in that**
on the left side and on the right side, the other miniplast splint (02) has positioning means (05) which are substantially adjustable in the longitudinal direction (X).

13. The occlusal splint arrangement (01) according to any of the claims 8 to 12,
**characterised in that**
the adjustment can be performed by means of an adjusting screw (08), an additional adjusting pin (36) for adjusting the adjusting screw (08) being arranged on the disassembling tool (31).

## Revendications

1. Système de gouttière occlusale (01), en particulier pour la thérapie de l'apnée du sommeil, comprenant une gouttière miniplast maxillaire (02) pouvant être disposée sur la rangée de dents maxillaire et une gouttière miniplast mandibulaire (12, 62) pouvant être disposée sur la rangée de dents mandibulaire, lesdites gouttières miniplast (02, 12, 62) étant réalisées avec des surfaces d'appui (04, 14, 64a) planes qui (04, 14, 64a) peuvent être mises en appui les unes contre les autres, une broche de centrage (15, 65) faisant saillie de la surface d'appui (14, 64b) étant respectivement ancrée dans la gouttière occlusale (12, 62) sur le côté gauche et sur le côté droit dans la région molaire, des moyens de positionnement (221, 22r, 72) remplaçables de gauche et de droite comprenant un évidement de centrage (25, 75) complémentaire à la broche de centrage (15, 65) respective, et étant fixés à la gouttière miniplast (12, 62) de manière remplaçable en venant en appui contre la surface d'appui (14, 64b), la position relative des gouttières miniplast (02, 12, 62) l'une par rapport à l'autre étant définie dans le sens longitudinal (X) et/ou dans le sens transversal (Y) par une complémentarité de forme entre les moyens de positionnement (22, 72) remplaçables et des moyens de positionnement (05, 07) de l'autre gouttière miniplast (02),
**caractérisé en ce que**
la broche de centrage (15, 65) comprend un ergot d'encliquetage (19, 69) et l'évidement de centrage (25, 75) comprend une cavité d'encliquetage (26, 76), ledit ergot d'encliquetage (19, 69) provoquant une fixation du moyen de positionnement (22, 72) remplaçable sur la gouttière miniplast (12, 62) par encliquetage dans la cavité d'encliquetage (26, 76), ladite broche de centrage (15, 65) comprenant une section de base (16, 66) moulée dans la gouttière miniplast (12, 62) et une section de fixation (17, 67) faisant saillie de manière rigide au-delà de la surface d'appui (14, 64), une section de connexion (18, 68) élastiquement déformable étant moulée à la section de fixation (17, 67) et un ergot d'encliquetage (19, 69) étant disposé à l'extrémité libre de la section de connexion (18, 68).

2. Système de gouttière occlusale (01) selon la revendication 1,
**caractérisé en ce que**
la broche de centrage (15, 65) est réalisée d'une seule pièce ; et/ou que la broche de centrage (15, 65) est faite d'une tôle métallique, en particulier par découpage au laser.

3. Système de gouttière occlusale (01) selon la revendication 1 ou 2,
**caractérisé en ce que**
la section de connexion (18, 68) élastiquement déformable s'étend à partir de l'extrémité éloignée de la surface d'appui (14, 64) de la section de fixation (17, 67) rigide le long de la section de fixation (17, 67) en direction de la surface d'appui (14, 64).

4. Système de gouttière occlusale (01) selon la revendication 3,
**caractérisé en ce que**
la section de connexion (18) élastiquement déformable est adjacente à la section de fixation (17) rigide et, en commençant par là, s'étend de manière courbée autour d'un oeil libre de 170° ± 10°, en particulier de 175° ± 5°.

5. Système de gouttière occlusale (01) selon la revendication 4,
**caractérisé en ce qu'**
à l'état non-encliqueté, une fente s'évase entre la section de fixation (17) et la section de connexion (18) à partir de l'extrémité de la section de fixation (17), en particulier à partir de l'oeil libre.

6. Système de gouttière occlusale (01) selon l'une quelconque des revendications 3 à 5,
**caractérisé en ce qu'**
à l'état encliqueté, une surface antérieure à la section de fixation (17) et une surface arrière à la section de fixation (18) s'étendent essentiellement parallèlement l'une à l'autre.

7. Système de gouttière occlusale (01) selon la revendication 3,
**caractérisé en ce qu'**
à l'état non-encliqueté, la section de connexion (68) se lève de plus en plus par rapport à la section de fixation (67) dans le sens transversal (Y) à partir de l'extrémité de la section de fixation (67).

8. Système de gouttière occlusale (01) selon l'une quelconque des revendications 1 à 7,
**caractérisé en ce que**
le moyen de positionnement (22, 72) remplaçable comprend au moins un évidement de démontage (27, 77), en particulier un évidement de démontage continu, dans lequel une broche de démontage (33) d'un outil de démontage (31) peut être insérée pour l'enlèvement du moyen de positionnement (22, 72) remplaçable de la gouttière miniplast (12, 62), ledit évidement de démontage (27, 77) étant orienté de manière transversale par rapport à l'évidement de centrage (25, 75), une broche de démontage (33) insérée provoquant un désencliquetage de l'ergot d'encliquetage (19, 69) de la cavité d'encliquetage (26, 76).

9. Système de gouttière occlusale (01) selon la revendication 8,
**caractérisé en ce que**
l'évidement de démontage (27, 77) coupe l'évidement de centrage (25, 75) dans la région de la cavité d'encliquetage (26, 76), en particulier que la cavité d'encliquetage (26, 76) est réalisée par une section de l'évidement de démontage (27, 77), une broche de démontage (33) insérée venant en appui contre l'ergot d'encliquetage (19, 69), provoquant ainsi un désencliquetage de l'ergot d'encliquetage (19, 69) de la cavité d'encliquetage (26, 76).

10. Système de gouttière occlusale (01) selon l'une quelconque des revendications 8 ou 9,
**caractérisé en ce que**
l'extrémité libre de la broche de démontage (33) a une coupe transversale qui se rétrécit.

11. Système de gouttière occlusale (01) selon l'une quelconque des revendications 8 à 10,
**caractérisé en ce qu'**
après l'insertion de la broche de démontage (33) dans l'évidement de démontage (27, 77), l'outil de démontage (31) peut venir en appui avec une surface de glissement (34) contre les surfaces d'appui (14, 64) de la gouttière miniplast (12, 62), le moyen de positionnement (22, 72) remplaçable étant écarté en le poussant de la broche de centrage (15, 65) en tournant l'outil de démontage (31) autour de l'axe central (35) de la broche de démontage (33) insérée.

12. Système de gouttière occlusale (01) selon l'une quelconque des revendications 1 à 11,
**caractérisé en ce que**
sur le côté gauche et sur le côté droit, l'autre gouttière miniplast (02) comprend des moyens de positionnement (05) ajustables essentiellement dans le sens longitudinal (X).

13. Système de gouttière occlusale (01) selon l'une quelconque des revendications 8 à 12,
**caractérisé en ce que**
l'ajustage peut être effectué en utilisant une vis de réglage (08), une broche de centrage (36) supplémentaire étant disposée à l'outil de démontage (31) pour l'ajustement de la vis de réglage (08).
